# EUROPEAN PATENT APPLICATION

(11) **EP 0 726 316 A2**
(43) Date of publication of application: **14.08.1996**
(21) Application number: 96300681.2
(22) Date of filing: 31.01.1996
(51) Int. Cl.: C12N 15/44, C07K 14/11, C12N 15/62, G01N 33/569

(54) **Expression of the non-structural protein NS1 of influenza virus and detection of anti-NS1 antibody in serum**

(30) Priority: 09.02.1995 GB 9502489
(71) Applicant: ANIMAL HEALTH TRUST, Newmarket, Suffolk CB8 7DW (GB)
(72) Inventor: Binns, Matthew, Ely, CAMBS. CB6 1BQ (GB); Birch-Machin, Ian, Cambridge, CAMBS CB4 2NR (GB)
(74) Representative: Davies, Jonathan Mark

(57) **Abstract**

The nucleotide sequence of the nonstructural protein NS1 of the influenza virus A/equine 2/Suffolk/89 was determined and found to be 97% identical to that of A/equine 2/Miami/63. A similar level of identity was shown for the deduced NS1 amino acid sequence. The NS1 gene was expressed, in its entirety and in part, as fusion proteins with glutathione S-transferase using, the pGEX-3X expression vector. Antibodies to NS1 protein were detected in serum samples from ponies experimentally infected in influenza virus, but not in animals vaccinated with whole inactivated virus or in unprimed control animals. The antigenic determinants(s) of NS1 protein appear to be located in the C-terminal half of the protein. The invention makes possible the use of NS1 protein as a diagnostic marker for influenza virus infection in the presence of high levels of circulating antibody to influenza haemagglutinin generated by recent vaccination. Use as a potential vaccine component is also possible. A recombinant fusion protein of glutathione S-transferase and the C-terminal half of NS1 (incorporating amino acid residues 105-230) was tested in an ELISA to detect influenza A virus infection. The detection rate of the ELISA compared to the methods used for the detection of anti-haemagglutinin antibodies was 60-79%. It was able to detect infection against a background of vaccination in samples from experimentally challenged animals and from training yards in which infection following vaccination was suspected.

## Description

The present invention relates to a non-structural protein NS1 of the influenza virus, especially NS1 of Equine Influenza A virus, and fusion proteins incorporating part of NS1. It also relates to the detection of anti-NS1 antibodies in a diagnostic test for influenza infection.

Infection of the equid respiratory tract by influenza A virus produces a number of symptoms including a febrile response, coughing, lethargy and a serous nasal discharge. In addition, opportunist bacterial infection can give rise to bacterial bronchopneumonia and other complications particularly in horses that are forced to exercise or are stressed in other ways. Virus detection can be carried out by isolation of the virus in embryonated hens eggs using material extracted from nasopharyngeal swabs (Burrows, 1968; Burrows et al, 1981). However, this method takes 3-4 days to complete, is dependent on the presence of viable virus and, in horses with subclinical or mild infection, may take several weeks (that is several egg passages) before sufficient virus is produced to permit identification (Mumford, 1990). An alternative method is to use an antigen capture ELISA which detects influenza nucleoprotein antigen in material extracted from nasopharyngeal swabs (Cook et al, 1988). This method is rapid, giving same day diagnosis on receipt of samples and is able to detect infection in horses shedding small amounts of virus. Moreover, it does not depend upon the infectivity of the sample which may be reduced during its transit to the laboratory. The nucleoprotein (NP) ELISA only detects the transient shedding of virus which during an acute infection may be between five and six days post infection (Cook et al, 1988). However in a subclinical infection which may be expected in a vaccinated population it may take time to identify the problem because symptoms are less acute and it may be too late to obtain a nasal swab or an acute serum sample. In addition the virus shedding stage will be of a shorter duration and at a lower level which may mean that the NP ELISA may not detect the virus because even with samples from an acute infection it is not 100% effective (Cook et al, 1988). Serologically, evidence of influenza infection is determined by haemagglutin inhibition (HI) or single radial haemolysis (SRH) assays on acute and convalescent samples to determine levels of serum anti-haemagglutinin antibody (Wood et al 1983).

Equine populations worldwide have undergone several influenza epizootics (see Livesay et al (1993) and references therein). Following the 1979 influenza outbreak in the U.K., vaccination was made mandatory for horses in training and other competition horses. This policy was believed to have contributed to the prevention of an epizootic until the last major outbreak in 1989 (Livesay et al, 1993). Repeated vaccinations lead to persistently high HI titres, such that following infection, HI titres may be boosted by less then four-fold thereby making it difficult to diagnose infection by serology. Animals may be vaccinated in the face of infection with the concomitant rise in HI titres due to both vaccination and infection.

Thus, there is currently a significant problem with existing methods for the detection of equine influenza because of the combination of mandatory vaccination of competition horses and the current use of fairly poor vaccines which do not give a very good level of protection from infection.

RNA segment 8 of influenza A viruses encodes two overlapping proteins which are thought to be present only in virus-infected cells (Lazarowitz et al, 1971; Skehel, 1972; Inglis et al, 1980). These proteins are referred to as NS1 and NS2 and are considered to be nonstructural proteins, although Richardson & Akkina (1991) detected NS2 in purified virus confirming similar earlier observations (Lamb et al, 1978; Akkina et al, 1984). NS1 is synthesized in large amounts early in infection and accumulates in the nucleus of infected cells, and is present in polysomal cell fractions (Dimmock, 1969; Lazarowitz et al, 1971; Compans, 1973); two independent signals for nuclear localization have been identified in the NS1 molecule (Greenspan et al, 1988). Furthermore, NS1 has also been found in association with cellular RNA in the form of paracrystalline inclusion bodies within the cytoplasm of infected cells (Yoshida et al, 1981; Shaw et al, 1982). Considerable heterogeneity with respect to molecular size, charge and phosphorylation has been demonstrated for several NS1 proteins. The extent of phosphorylation appears to be characteristic of particular subtypes (Petri et al, 1982). Some NS1 proteins have been shown to have large carboxyl terminal deletions which apparently do not alter the functional integrity of the protein (Parvin et al, 1983; Norton et al, 1987). The function of NS1 protein during viral multiplication remains unclear, although a possible regulatory role in viral replication has been suggested from studies of temperature-sensitive mutants of NS1 (Wolstenholme et al, 1980; Koennecke et al*,* 1981). It has been proposed that NS1 protein stimulates the translation of the Ml protein (Enami et al, 1994) but its effect may rather be through the regulation of mRNA nuclear export (Qui and Krug., 1994; Qian et al, 1994). Conversely, Huang et al (1990) have shown that NS1 protein was not required for replication and expression of viral ribonucleoprotein.

Antigenic analyses of purified NS1 proteins from several subtypes has indicated cross-reactivity among all the influenza A virus strains tested (Shaw et al, 1982; Brown et al, 1983; Young et al, 1983), although antigenic drift (from the earliest to the most recent virus isolate) was detected (Shaw et al, 1982). The NS1 gene from various human influenza virus strains has been cloned (Baez et al, 1980; Krystal et al, 1983) and the protein successfully expressed in Escherichia coli (Young et al, 1983; Hatada et al, 1992) and vaccinia (Smith et al, 1987).

The nonstructural proteins of Yellow Fever virus (Schlesinger et al, 1986; Cane & Gould, 1988) and Dengue viruses (Schlesinger et al, 1987; Qu et al, 1993) have been used as immunogens with some protective success, whilst antibodies to nonstructural proteins have been shown to be potential diagnostic markers for early hepatitis C infection (Inoue et al, 1992) and foot and mouth disease virus infection (Nietzert et al, 1991).

The nucleotide sequence of the nonstructural protein NS1 of the influenza virus A/equine 2/Suffolk/89 was determined and found to be 97% identical to that of A/equine 2/Miami/63. A similar level of identity was shown for the deduced NS1 amino acid sequence.

In a first aspect of the invention, the NS1 gene was expressed in its entirety, and in part as fusion proteins with glutathione S-transferase (GST).

Antibodies to NS1 protein were detected in serum samples from ponies experimentally infected with influenza virus, but not in animals vaccinated with whole inactivated virus or in unprimed control animals. The antigenic determinant(s) of NS1 protein appear to be located in the C-terminal half of the protein. The NS1 protein only appears in infected cells (Dimmock, 1969; Lazarowitz et al, 1971) and is not incorporated into viral particles and therefore horses routinely vaccinated with whole inactivated virus will not be exposed to this antigen. The invention makes possible the use of NS1 protein as a diagnostic marker for influenza virus infection in the presence of high levels of circulating antibody to influenza haemagglutinin generated by recent vaccination.

In a second aspect the invention provides for the use of NS1 protein from an equine influenza virus in its entirety or in part as a fusion protein with glutathione S-transferase (GST) in a diagnostic test for the rapid detection of equine influenza infection even following recent vaccination.

The invention is exemplified hereafter with reference to the cloning, sequencing and expression of the equine influenza NS1 protein and its use either complete or as part of a fusion protein in a diagnostic test for equine influenza.

In the following:
Figure 1(a) shows the nucleotide sequence (NS1 : SEQ.ID 6) of the non-structural protein of equine influenza A virus;
Figure 1(b) shows the hydrophobicity profile of NS1;
Figure 2 shows the SDS-PAGE of expressed NS1 fusion proteins;
Figure 3(a) shows an immunoblot analysis of NS1 protein with pre-challenged serum from a vaccinated animal;
Figure 3(b) shows an immunoblot analysis of NS1 protein with post-infection serum;
Figure 4(a) shows HI titres of sera of horses exposed to equine influenza;
Figure 4 (b) shows NS1 ELISA of sera of horses exposed to equine influenza.

### Description of the Invention

### Materials and methods

### Viral RNA isolation and cloning

Equine influenza virus A/equine 2/Suffolk/89 (H3N8) was grown in embryonated chicken eggs and purified by gradient centrifugation as previously described (Cook et al, 1988). Viral RNA was extracted according to the method of Binns et al (1992). Reverse transcription (Chirnside & Spaan, 1990) was carried out using 5µl of RNA with the oligonucleotide 5'-CGGGATCCCCATGGATTCCAACACTGTGTCA-3' (primer 21/91:SEQ.ID.1). Polymerase chain reaction (PCR) was then performed using primer 21/91 and primer 22/91 (S'CGGGATCCTCAAACTTCTGACTCAATTGT-3':SEQ.ID.2) as described (Binns et al, 1992) except that the amplification consisted of 30 cycles of 1 min at 94°C, 2 min at 55°C, and 1.5 min at 72°C. The DNA produced was genecleaned according to the manufacturer's instructions (Bio 101, La Jolla, CA, U.S.A.) and then digested with BamHI, sites for which had been included in primers 21/91 and 22/91 to facilitate the cloning procedure. The BamHI sites were preceded by two nucleotides to improve digestion efficiency and followed by 23 and 21 NS1-specific nucleotides (Nakajima et al, 1990) in 21/91 and 22/91 respectively. The digested DNA was ligated into BamHI-cleaved pGEX-3X or pUC 13 (Pharmacia, Uppsala, Sweden). Following transformation into E. coli TG1, colonies were blotted onto nitrocellulose filters (Hybond N; Amersham Life Sciences, Amersham, U.K.) according to the supplier's instructions and screened for the presence of NS1 insert using a 32P-labelled NS1 fragment (Feinberg & Vogelstein, 1984) isolated from low melting temperature agarose. DNA was prepared from positive recombinants using the Magic Miniprep System (Promega, Madison, WI, U.S.A.) and digested with HindIII and PstI to check for the correct orientation of the NS1 insert.

### Cloning of Portions of the NS1 gene

The NS1 gene was also cloned into two approximate halves, referred to as NS1-N and NS1-C. This was achieved by ligating two BamHI-digested PCR products into BamHI-cleaved pGEX-3X as described above. PCR was performed using template DNA prepared from the recombinant NS1 clone in the PUC vector. The two primer pairs used were primers 22/91 and 129/92 (5'-CGGGATCCTCATGCCCAAGCAG-3':SEQ.ID.3), and primers 21/91 and 130/92 (5'-CGGGATCCTGATGTTCTTATCCA-3'sEQ.ID.4), respectively. Positive recombinants were screened as before and correct orientation determined by HindIII/PstI or BspHI/PstI digestion of the plasmid DNA.

### Sequencing of the NS1 gene and constructs

Recombinants of interest were sequenced using the dsDNA Cycle Sequencing System (Gibco BRL, Paisley, U.K.). The primers used were primer 22/91 (above), primer 36/91 (S'GCGACCATCCTCCAA-3':SEQ.ID.5; specific for pGEX vectors), as well as two primers based on primers 129/92 and 130/92 but lacking the BamHI cloning "tag". The annealing temperatures were 60°C, 55°C, 48°C and 40°C, respectively.

### Production and purification of NS1 fusion protein

Essentially this was as described by Frangioni and Neel (1993). Briefly, recombinant bacteria were grown overnight at 37°C in 2ml NZY medium (10 g NZ amine A (Sigma, Poole, U.K.), 5 g yeast extract, 5 g NaCl, pH 7.5), supplemented with 50µg ampicillin (Sigma) per ml, and then 50µl was added to 2ml NZY medium + 50µg ampicillin per ml. After 2h in an orbital shaker at 37°C, the culture was induced by the addition of isopropyl-thio-βgalactopyranoside (Sigma) to a final concentration of 1mM. The incubation was continued for 2-3h. Bacteria were pelleted, washed once with 200µg of ice-cold STE (10mM Tris pH 8.0, 150mM NaCl, 1mMEDTA) and resuspended by repeated pipetting in 133µl of STE containing 100µg/ml of lysozyme (freshly made). After incubation on ice for 15min diothiothreitol (DTT) was added to a final concentration of 5mM and sarkosyl to a final concentration of 1 %. The bacteria were vortexed for 5s and then lysed by sonication in a sonic water bath (QH Kerry, Kerry Ultrasonics Ltd.) for 1 min. The lysate was clarified by centrifuging for 15min in a microfuge, full speed, at 4°C and the supernatant was transferred to a new eppendorf tube. Triton X 100 (BDH Chemicals Ltd., Poole, U. K.) was added to a final concentration of 1 % and the lysate was vortexed for 5s. The supernatant was then mixed with 200µl of glutathione Sepharose 4B (Pharmacia) suspension (50% in PBS (150mM NaCl, 16mM Na2HPO4, 4mM NaH2P04, pH7.5), washed by repeated centrifugation and resuspension (3 times) for 15min. The unbound material was removed following centrifugation (full speed, l0s) and the gel was washed twice with lml PBS for 5 min on a rotator (30rpm). The bound fusion protein was eluted with lml elution buffer (75mM Hepes, pH7.4, 150mM NaCl, 10mM reduced glutathione (Sigma), 5mM DTT, 0.1% Triton X100) and stored at 20°C after the addition of glycerol to a final concentration of 10% (v/v).

### Sodium dodecyl sulphate polyacrylamide gel electrophoresis (SDS-PAGE) and immunoblotting

Following expression of the recombinant NS1 protein, bacteria were pelleted in a microfuge, resuspended in sample buffer and electrophoresed in slab gels (12.5 %) in the presence of SDS according to the method of Laemmli (1970). Proteins were transferred electrophoretically to nitrocellulose sheets as previously described (Towbin et al, 1979). Prestained standards (Bio-Rad Laboratories, Hemel Hempstead, U.K.) were used as molecular weight markers for all blots. Following protein transfer, blots were blocked overnight at 4°C in 5% goat serum (Sera Labs, Crowley Down, Sussex) in PBS containing 0.1% (v/v) Tween 20 (Sigma) (PBST). Blots were washed three times in PBST at 37°C (5 min/wash) and then incubated at 37°C with serum samples diluted 1:2500 in PBST containing 5% goat serum (Dilution Buffer) for 90min. Each blot was washed 3 times in PBST at 37°C (5min/wash) and then incubated with goat anti-horse IgG-biotin conjugate (Dynatech Lab Ltd, Daux Road, Billingshurst, Sussex) diluted 1: 1000 in Dilution Buffer for 90min at 37°C. Following 3 washes in PBST at 37°C (5min/wash) each blot was incubated with streptavidin-peroxidase conjugate (Dynatech Lab Ltd) diluted 1:1000 in Dilution Buffer for 30min at room temperature. After washing 6 times in PBST (room temperature, 5min/wash) the blots were developed with either 3',3' diaminobenzidine and 3 % H₂O₂ or Amersham's ECL detection system.

### Experimental challenge of ponies with equine influenza

Ponies vaccinated with inactivated whole virus (Wood et al 1983; Mumford, 1992) and unvaccinated control animals were infected with A/equine 2/Newmarket/79 or A/equine 2/Suffolk/89 using nebulised aerosols of allantoic fluid as described previously (Mumford et al, 1990). Serum samples collected before and fourteen days after challenge were tested for the presence of anti-haemagglutinin antibodies using the HI and/or SRH tests (Burrows et al, 1977; Wood et al, 1983) and anti-NS1 antibodies by immunoblotting (above) and ELISA (below).

### ELISA

The field sera used in this study were either submitted to the diagnostic service of the Animal Health Trust or obtained from Professor K.F. Shortridge (Microbiology Dept., Hong Kong University) and Mr K.L. Watkins (The Royal Hong Kong Jockey Club).

Microtitre plates (Dynatech immulon III) were incubated at 4°C overnight with 100µl (per well) of a solution containing 500-1500ng of purified GST or NS1-C fusion protein in 0.05M carbonate buffer (pH 9.6). The coated plates were washed three times in PBSJ (150mM NaCl, 16mM NA2HPO4, 4mM NaH2PO4, 0.1% v/v Tween 20, pH 7.5) then blocked for 90 minutes at 37°C with 100µl (per well) of Dilution Buffer (PBST + 5% goat serum). The ELISAs were performed by incubating aliquots of horse serum diluted 1:100 in Dilution Buffer for 90 minutes at 37°C in a humid chamber. After three washes with PBST, 100µl of goat anti-horse IgG-biotin conjugate diluted 1:1000 in Dilution Buffer was added to the wells and the plates were incubated at 37°C for 90 minutes. The plates were washed three times in PBST and then 100µl of Streptavidin-peroxidase conjugate diluted 1:1000 in Dilution Buffer was added to the wells and the plates were incubated at room temperature for 30 minutes. Following three washes with PBST, 100µl of O-Phenylenediamine dihydrochloride (Sigma, 10µg/ml) in 0.05M phosphate citrate buffer and 0.03% sodium perborate (Sigma) were added to each well and the plates were incubated at room temperature for 10 minutes. The reaction was stopped with 4M sulphuric acid (50pl/well) and the absorbance at 490 nm was read with a microplate reader (Molecular Devices).

### RESULTS

### Nucleotide sequence of the Suffolk/89 NS1 gene

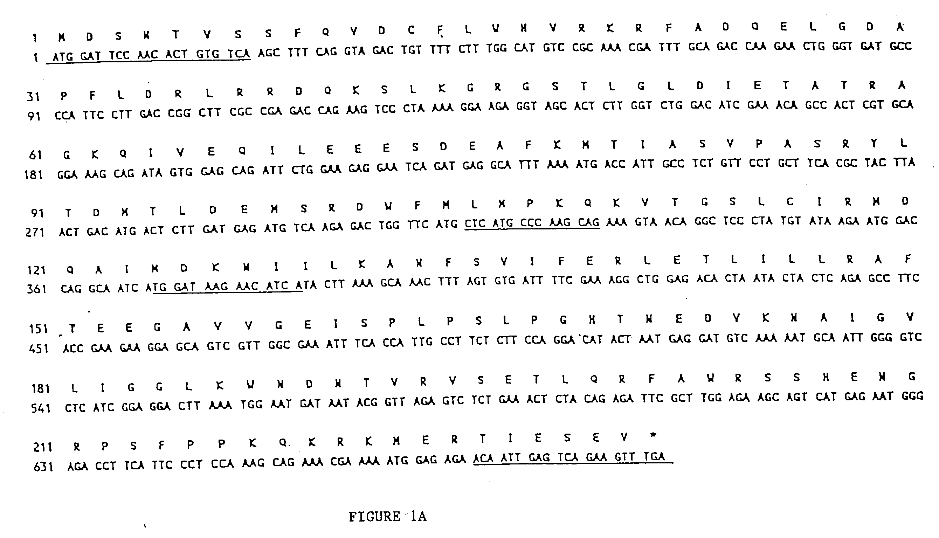

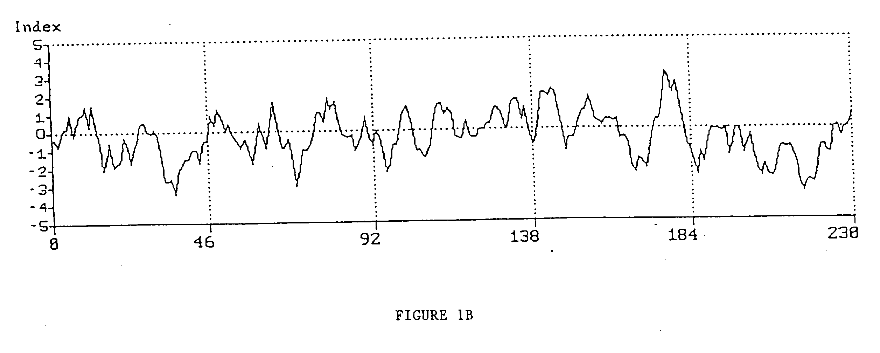

Cloning of the Suffolk/89 NS1 gene was achieved using reverse transcription and PCR with primers based on the nucleotide sequence of the A/equine2/Miami/63 (H3N8) NS 1 gene (Nakajima et al, 1990). The nucleotide sequence of the Suffolk/89 NS1 gene was determined using two independently isolated clones to identify any potential artefacts generated during the PCR amplification. Both clones possessed identical sequences which are presented in Fig. 1a. Both sequences start at the initiating methionine since reverse transcription was performed with a primer based on the first seven codons of the Miami/63 NS1 gene (Nakajima et al, 1990). Primers used for cloning (minus their BamHI-cloning tags) and sequencing are underlined. In comparison to the Miami/63 NS1 gene, the nucleotide sequence was found to be 97 % identical as was the deduced amino acid sequence. The clones designed to express the NS1 protein in two approximate halves, designated NS1-N and NS1-C, were sequenced and shown to be in the correct reading frame coding for amino acid residues 1 - 128 and 105 - 230, respectively. A hydrophobicity profile of the deduced amino acid sequence of the Suffolk/89 NS1 protein is shown in Fig. 1b. The hydrophobicity profile of the NS1 protein is presented as a plot of the hydropathic index (arbitrary units) versus amino acid residue number (Kyte & Doolittle, 1982).

### Expression of NS1 fusion proteins

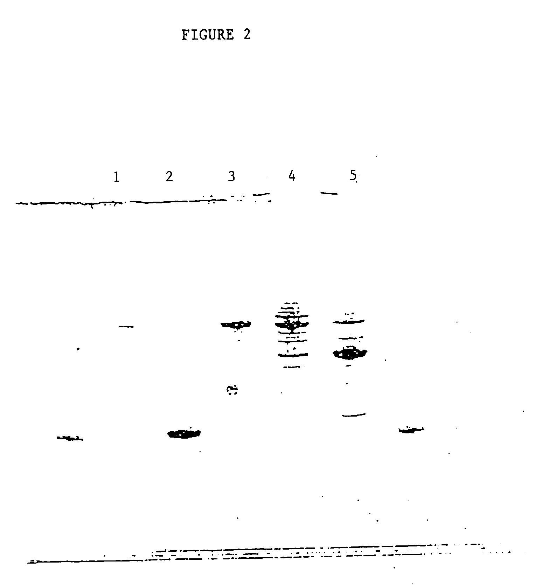

Following induction, cells were pelleted and analysed directly by SDS-PAGE as described in the Methods section. Results are shown in Figure 2 which shows the following: Lane 1, uninduced pGEX-3X; lane 2, induced pGEX-3X (GST); lane 3, NS1; lane 4, NS1-N; lane 5, NS1-C.

SDS-PAGE of induced bacterial cell pellets indicated good levels of expression of the various fusion proteins. The molecular weights compared well to the expected values; the predicted amino acid sequence of NS1 produces a protein of molecular weight 26,178 Da, thus yielding fusion proteins of approximately 52.6 kDa (NS1), 41.1 kDa (NS1-N) and 40.7 kDa (NS1-C). Attempts to purity the NS1 fusion protein proved largely unsuccessful although a small amount was obtained from the glutathione affinity column. This poor recovery was attributed primarily to the low solubility of the fusion protein following lysozyme, sonication or freeze/thaw treatments (results not shown). Cloning smaller portions of the NS1 protein (NS1-N and NS1-C) unfortunately did not produce more soluble fusion proteins following lysozyme treatment and lysis in Triton xl00. However Frangioni and Neel (1993) have shown that sarkosyl can be used to solubilise most GST fusion proteins and that subsequent treatment with non-ionic detergent allows the sarkosyl solubilised GST fusion protein to bind with high affinity to glutathione agarose. Therefore the smallest portion of the NS1 protein (NS1-C) was expressed in E. coli as a fusion protein with GST and purified in the presence of sarkosyl. The addition of Triton X-100 allowed binding of the GST fusion protein (data not shown) to glutathione agarose and purification of the NS1-C protein. Protein estimations (Sigma) showed that the yield was of the order of 2mg/ml purified protein.

### Immunoblot analysis of NS1 fusion proteins

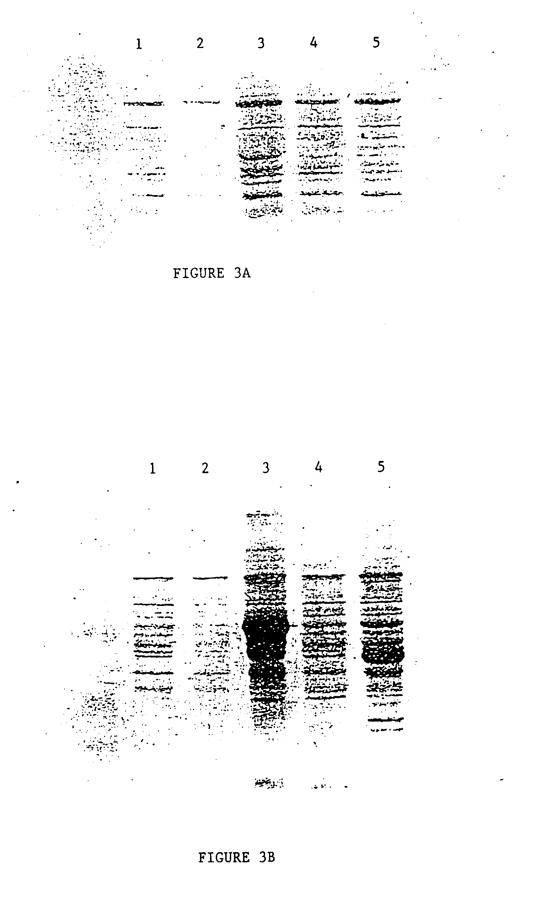

Immunologic analysis of NS1 protein with serum from influenza challenged ponies was carried out using immunoblotting. Figure 3 shows the results from one horse which are representative of those obtained for the other animals in this experimental challenge experiment. In Figure 3 the lanes are as follows: lane 1, uninduced pGEX-3X; lane 2, induced pGEX-3X (GST); lane 3, NS1; lane 4, NS1-N; lane 5, NS1-C. It can be seen in Fig3 (a) that only minor non-specific reactions were observed with the high titre pre-challenged sera from vaccinated animals (tested positive for serum influenza anti-haemagglutinin antibodies). Positive reactions to the NS1 fusion protein (and also to NS1-C) were only observed with the post infection serum samples (see Fig3(b)). No responses were observed against NS1-N or GST protein itself. Control blots in the absence of any serum gave no reactions to the fusion protein (not shown).

### ELISA

To determine the detection efficiency and background readings for the ELISA, sera from experimentally challenged animals were tested for the presence of anti-NS1 antibodies. In all cases both pre-challenge and post-challenge sera were tested against purified fusion protein and separately against purified glutathione S-transferase (GST) to establish the extent to which the sera binds to GST. These studies indicated a detection efficiency of 60% with a positive result being an A490 reading greater than two standard deviations above the mean of the pre-challenge sera results, that is ≥0.1. In all ELISAs the GST reading was subtracted from the reading derived from the fusion protein.

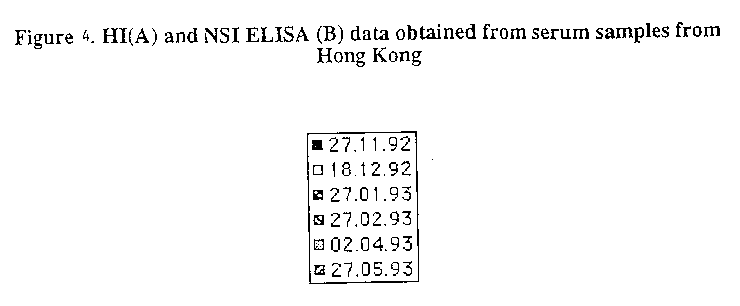

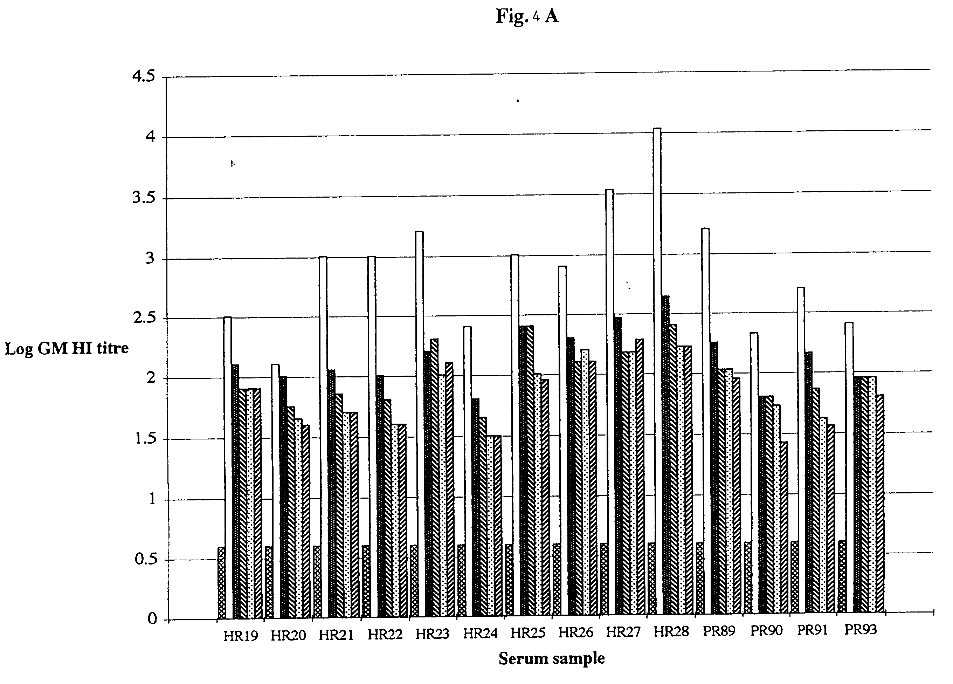

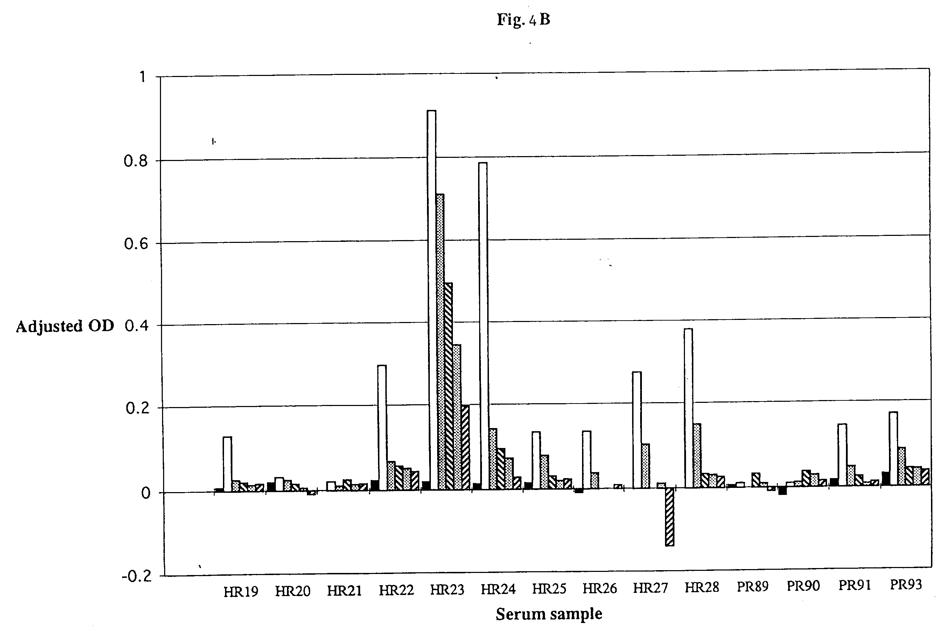

To establish whether the ELISA could detect infection in the field, samples were obtained from horses recently involved in an outbreak in Hong Kong. Fifteen non-vaccinated naive riding horses/ponies were imported from Australia just prior to an influenza outbreak in November 1992 and were exposed to equine influenza at their stables. They were sampled on six separate occasions and HI titres (Figure 4(a)) indicated seroconversion had taken place in all fourteen animals. Furthermore, there was a decline in antibody titre after the first month of infection, followed by a slow decrease over the following three months. When the NS1 ELISA was performed on the convalescent samples, infection was detected in 11 out of 14 samples, corresponding to a detection rate of 79% (Figure 4(b)). Within one month the serum from only four horses were NS1 ELISA-positive and after two months only one horse was tested positive. This indicates that the duration of antibodies against NS1 is short lived being between 1-2 months. In a separate analysis of experimentally infected animals the duration of the response to NS1 was between 2-3 months post infection (data not shown). This makes detection of NS1 antibodies more useful as a diagnostic marker for influenza as the relatively short lived response means that positive responses indicate recent infection and allows the detection of subsequent reinfection.

In order to be practically useful as a marker for influenza infection in the field, the detection of NS1 has to be performed against a background of vaccination. The sera in the previously described experiments were obtained from non-vaccinated horses/ponies. Therefore serum from three vaccine trials conducted at the Animal Health Trust were tested for the presence of NS1 antibodies. The animals had been vaccinated and then challenged with influenza and in addition a group of control animals which were not vaccinated were also challenged. Pre and post-challenge sera were taken and tested in the NS1 ELISA. For the control animals a positive NS1 ELISA result was obtained in post-challenge sera from 6 out of 10 animals (i.e. 60%) which is similar to the 79% detection rate described above for naive animals. For the vaccinated animals all pre-challenge sera were negative demonstrating that as predicted vaccination does not generate an NS1 response. Following challenge of the vaccinated animals, the clinical signs and virus excretion were significantly reduced compared to the unvaccinated control animals and the NS1 ELISA was positive for 5 out of 19 animals (26%). Many of the vaccinated animals were solidly protected from infection such that we would not have expected to observe anti-NS1 antibodies. However, the fact that 26% were NS1 ELISA positive demonstrates that the NS1 ELISA is capable of detecting influenza infection under the cover of vaccination.

The ELISA was then used to investigate an influenza outbreak in a training yard from samples submitted to the Animal Health Trust diagnostic service in an attempt to evaluate how effective it would be.

Acute and convalescent samples were submitted and HI tests were performed on each. From the first yard (Table 1) 7/16 horses (C, D, F, G, H, K, L) seroconverted indicating that they had become infected. However seroconversion was not evident in the other nine samples. Of these, four (B, J, N, P) were NS1 ELISA-positive, indicating that they had also been infected.

### Discussion

We have described the use of a recombinant NS1 GST fusion protein to detect antibodies specific for influenza infection by ELISA. The test proved to be highly specific and, although not detecting all infections, it was capable of identifying infection in the absence of seroconversion as measured by HI. In addition, the production of antibodies against NS1 is short lived which enables subsequent re-infection to be easily detectable. At present there is no rapid diagnostic marker available to detect equine influenza virus infection under the cover of vaccination. The use of the NS1 recombinant protein in an ELISA provides a rapid (sane day) , relatively selective diagnostic procedure based upon a protein where expression and purification is simple. In particular, in batch testing of yards and when paired samples are not available the ELISA will be of great practical importance.

The deduced amino acid sequence of the Suffolk/89 NS1 protein revealed a very high degree of homology with another sequenced NS1 protein from the same H3N8 subtype, A/equine 2/Miami/63 influenza virus. Furthermore, it also appears to confirm the suggestion of "the existence of constraints on the amino acid changes of the NS1 protein in evolution" (Nakajima et al, 1990) This conservation tends to imply an important functional role as has been suggested previously (Wolstenholme et al, 1980; Koennecke et al, 1981). Such a high degree of conservation also allows this protein to be utilised as a marker for viral infection. Since competition horses are routinely vaccinated with whole inactivated virus, and NS1 protein only appears in infected cells (Dimmock, 1969; Lazarowitz et al, 1971), vaccinated animals will not be exposed to this antigen whereas those suffering a viral infection will. This protein would therefore appear to be a suitable candidate for a differential diagnostic marker, capable of distinguishing infected from vaccinated horses if antibodies to NS1 protein are produced during influenza infection. It would also be particularly useful in cases where only a single convalescent serum sample is available. NS1 protein is known to be antigenic (Shaw et al, 1982; Brown et al, 1983) and the results in Figure 3(b) clearly indicate that antibodies to NS1 protein are present in the serum of influenza challenged ponies. The specificity of these reactions was further confirmed by the lack of response to the GST portion of the fusion protein (lane 2). Cross-reactivity was also observed since positive reactions were obtained with serum from ponies exposed to the Newmarket/79 strain, further supporting the high conservation of NS1 proteins. As far as we are aware this is the first report of antibodies to NS1 being identified following influenza infection. The finding that the purification of the fusion protein was difficult may be explained, at least in part, by the hydrophobic nature of the NS1 protein (see Fig. 1b). NS1 protein is predominantly hydrophobic with only the final one-fifth of the protein being hydrophillic. It would appear that in combination with GST, which itself is quite soluble, the hydrophobicity of these fusion proteins may lead to the formation of insoluble inclusion bodies, thus preventing the release of the vast majority of the protein upon cell lysis. The occurrence of insoluble GST fusion proteins has been reported previously (Smith & Johnson, 1988) with the presence of strongly hydrophobic regions the major cause of the insolubility. However the inclusion of sarkosyl in the purification method enabled the production of soluble fusion protein.

The antigenic determinant(s) of NS1 protein are situated in the C-terminal half of the protein as judged by the responses to NS1-N and NS1-C (Fig. 3). Interestingly, a cytotoxic T lymphocyte epitope has been identified on the NS1 protein of human influenza A/PR/8/34 virus corresponding to residues 152-160 (Cossins et al, 1993). Highly homologous regions are present at the same position in the C-terminal portions of both the Miami/63 and Suffolk/89 NS1 proteins, as well as many other influenza NS1 proteins (Nakajima et al, 1990), clearly indicating that these proteins should be capable of eliciting a similar immune response.

The usefulness of the NS1 protein as a diagnostic marker of influenza infection depends upon the duration of the antibody response to it following the initial infection. A prolonged response would obviously limit its use diagnostically. Results indicate it is short-lived, as is the case for anti-haemagglutinin antibodies (Hannant et al, 1988).

**Table 1.**

| HI and ELISA data obtained from serum samples submitted to the AHT diagnostic service. | | | | | | |
|---|---|---|---|---|---|---|
| Horse | HI | HI | Seroconversion | Adjusted ELISA A₄₉₀ | | ELISA +/- |
| A | 1024 | >1024 | - | -0.031 | -0.005 | - |
| B | 1024 | 1024 | - | 0.113 | 0.148 | + |
| C | 128 | 1024 | + | 0.018 | 0.016 | - |
| D | 128 | 1024 | + | 0.003 | 0.517 | + |
| E | 32 | 512 | - | 0.001 | 0.035 | - |
| F | 128 | 512 | + | 0.246 | 2.271 | + |
| G | 64 | 256 | + | 0.083 | 0.660 | + |
| H | 32 | 128 | + | 0.016 | 0.030 | - |
| I | 512 | 1024 | - | -0.009 | -0.019 | - |
| J | >1024 | >1024 | - | 0.214 | 0.136 | + |
| K | 256 | 1024 | + | 0.329 | 0.278 | + |
| L | 128 | 512 | + | 0.008 | 0.047 | - |
| M | 128 | 128 | - | -0.010 | -0.004 | - |
| N | 512 | >1024 | - | 0.172 | 0.234 | + |
| O | 256 | 512 | - | -0.019 | 0.015 | - |
| P | 1024 | 512 | - | -0.003 | 0.199 | + |

### REFERENCES

AKKINA, R. K., CHAMBERS, T. M. &NAYAK, D. P. (1984) Expression of defective-interfering influenza virus-specific transcripts and polypeptides in infected cells.

Journal of Virology 51, 395-403.

BAEZ, M., TAUSSIG, R., ZAZRA, J. J., YOUNG, J. F., PALESE, P., REISFELD, A. & SKALKA, A. (1980) Complete nucleotide sequence of the influenza A/PR/8/34 virus NS gene and comparison with the NS genes of the A/Udorn/72 and A/FPV/Rostock/34 strains. Nucleic Acids Research 8, 5854-5858.

BINNS, M. M., DALY, J. M., CHIRNSIDE, E. D., MUMFORD, J. A., WOOD, J. M., RICHARDS, C. M. & DANIELS, R. S. (1992) Genetic and antigenic analysis of an equine influenza H3 isolate from the 1989 epidemic. Archives of Virology, 130: 33-43.

BROWN, L. E., HINSHAW, V. S. & WEBSTER, R. G. (1983) Antigenic variation in the influenza A virus nonstructural protein, NS1. Virology 130, 134-143.

BURROWS, (1968) Laboratory diagnosis of some virus infections of the upper respiratory tract of the horse. Equine Vet J.,1,32-38.

BURROWS, R., SPOONER, P. R. & GOODRIDGE, D. (1977) A three-year evaluation of four commercial equine influenza vaccines in ponies maintained in isolation. Developments in Biological Standards 39, 341-346.

BURROWS, R, DENYER, M. GOODRIDGE, D. and HAMILTON, F. (1981) Field and laboratory studies of equine influenza viruses isolated in 1979. Vet. Rec., 109, 353-356.

CANE, P. A. & GOULD, E. A. (1988) Reduction of yellow fever virus mouse neurovirulence by immunization with a bacterially synthesized nonstructural protein (NS1) fragment. Journal of General Virology 69, 1241-1246.

CHIRNSIDE, E. D. & SPAAN, W. J. M. (1990) Reverse transcription and cDNA amplification by the polymerase chain reaction of equine arteritis virus (EAV). Journal of Virological Methods 30, 133-140.

COMPANS, R. W. (1973) Influenza virus proteins. 11. Association with components of the cytoplasm. Virology 51, 56-70.

COOK, R.F., SINCLAIR, R. and MUMFORD, J.A. (1988) Detection of influenza nucleoprotein antigen in nasal secretions from horses infected with A/equine influenza (H3N8) viruses. J. Virol.

Meth., 20, 1-12.

COSSINS, J., GOULD, K.G., SMITH, M., DRISCOLL, P. & BROWNLEE, G. G. (1993) Precise prediction of a K^{k}-restricted cytotoxic T cell epitope in the NS1 protein of influenza virus using an MHC allele-specific motif. Virology 193, 289-295.

DIMMOCK, N. J. (1969) New virus-specific antigens in cells infected with influenza virus. Virology 39, 224-234.

ENAMI, K., SATO, T.A., NAKADA, S. & ENAMI, M. (1994) Influenza virus NS1 protein stimulates translation of the M1 protein. Journal of Virology 68 1432-1437.

FEINBERG, A. P. & VOGELSTEIN, B. (1984) A technique for radiolabelling DNA restriction endonuclease fragments to high specific activity. Analytical Biochemistry 137, 266-267.

FRANGIONI, J. V. & NEEL, B. G. (1993) Solubilization and Purification of Enzymatically Active Glutathione S-Transferase (pGEX) Fusion Proteins. Analytical Biochemistry 210: 179-187.

GREENSPAN, D., PALESE. P. & KRYSTAL, M. (1988) Two nuclear location signals in the influenza virus NS1 nonstructural protein. Journal of Virology 62, 3020-3026.

HANNANT,D., MUMFORD, J.A. & JESSETT,D.M. (1988) Duration of circulating antibody and immunity following infection with equine influenza virus. Veterinary Record 122: 125-128.

HATADA, E., TAKIZAWA, T. & FUKUDA, R. (1992) Specific binding of influenza A virus NS1 protein to the virus minus-sense RNA in vitro. Journal. of General Virology 73, 1725.

HUANG, T.-S., PALESE, P. & KRYSTAL, M. (1990) Determination of influenza virus proteins required for genome replication. Journal of Virology 64, 5669-5673.

INGLIS, S. C., GETHING, M. J. & BROWN, C. M. (1980) Relationship between the messenger RNAs transcribed from two overlapping genes of influenza virus. Nucleic Acids Research, 8, 3575-3589. INOUE, Y., SUZUKI, R., MATSUURA, Y., HARADA, S., CHIBA, J., WATANABE, Y., SAITO, 1. & MIYAMURA, T. (1992) Expression of the amino-terminal half of the NS1 region of the hepatitis C virus genome and detection of an antibody to the expressed protein in patients with liver disease. Journal of General Virology 73, 2151-2154.

KOENNECKE, I., BOSCHEK, C. B. & SCHLOLTISSEK, C. (1981) Isolation and properties of a temperature-sensitive mutant (ts 412) of the influenza A virus recombinant with a ts lesion in the gene coding for nonstructural protein. Virology 110, 16-25.

KRYSTAL, M., BUONAGURIO, D., YOUNG, J. F. & PALESE, P. (1983) Sequential mutations in the NS genes of influenza virus field strains. Journal of Virology 45, 547-554.

KYTE, J. & DOOLITTLE, R. F. (1982) A simple method for displaying the hydropathic character of a protein. Journal of Molecular Biology 157, 105-103020.

LAEMMLI, U.K. (1970) Cleavage of structural proteins during the assembly of the head of bacteriophage T4. Nature (London.) 227, 680-685.

LAMB, R. A., ETKIND, P. R. & CHOPPIN, P. W. (1978) Evidence for a ninth influenza virus polypeptide. Virology 91, 60-78.

LAZAROWITZ, S. G., COMPANS, R. W. & CHOPPIN, P. W. (1971) Influenza virus structural and nonstructural proteins in infected cells and their plasma membranes. Virology 46, 830-843.

LIVESAY, G. L., O'NEILL, T., HANNANT, D., YADAV, M. P. & MUMFORD, J. A. (1993) The outbreak of equine influenza (H3N8) in the U.K. in 1989 and the use of antigen capture ELISA in the diagnosis of acute infection. Veterinary Record, 133: 515-519.

MUMFORD,J.A. (1992) Progress in the control of equine influenza. In Plowright,W., Rossdale,P.D. and Wade,J.F. eds Equine Infectious Diseases VI: Proceedings of the Sixth International Conference, R&W Publications, Newmarket, England, p207-217.

MUMFORD, J.A., HANNANT, D. & JESSETT, D.M. (1990) Experimental infection of ponies with equine influenza (H3N8) viruses by intranasal inoculation or exposure to aerosols. Equine Veterinary Journal 22, 93-98.

MUMFORD, J.A. (1990) The diagnosis and control of equine influenza. Proc. Am. Assoc. Equine Pract., 36, 377-385.

NAKAJIMA, K., NOBUSAWA, E. & NAKAJIMA, S. (1990) Evolution of the NS genes of the influenza A viruses. 11. Characteristics of the amino acid changes in the NS1 proteins of the influenza viruses. Virus Genes 4, 15-26.

NIETZERT, E., BECK, E., DE MELLO, P. A., GOMES, 1. & BERGMANN, I. E. (1991) Expression of the aphthovirus RNA polymerase gene in Escherichia coli and its use with other bioengineered nonstructural antigens in detection of late persistent infections. Virology 184, 799-804.

NORTON, G. P., TANAKA, T., TOBITA, K., NAKADA. S., BUONAGURIO, D. A., GREENSPAN, D., KRYSTAL, M. & PALESE. P. (1987) Infectious influenza A and B virus variants with long carboxyl terminal deletions in the NS1 polypeptides. Virology 156, 204-213.

PARVIN, J. D., YOUNG, J. F. & PALESE, P. (1983) Nonsense mutations affecting the lengths of the NS1 nonstructural proteins of influenza A virus isolates. Virology 128, 512-517.

PETRI, T., PATTERSON, S. & DIMMOCK, N. J. (1982) Polymorphism of the NS1 proteins of type A influenza virus. Journal of General Virology 61, 217-231.

QIAN,X-Y., ALONSO-CAPLEN,F., & KRUG,R.M. (1994) Two functional domains of the influenza virus NS1 protein are required for regulation of nuclear export of mRNA. Journal of Virology 68: 2433-2441.

QIU, Y., & KRUG, R.M. (1994) The influenza virus NS1 protein is a poly(A) -binding protein that inhibits nuclear export of mRNAs containing poly(A). Journal of Virology 68 2425-2432.

QU, X., CHEN, W., MAGUIRE, T. & AUSTIN, F. (1993) Immunoreactivity and protective effects in mice of a recombinant dengue 2 Tonga virus NS1 protein produced in a baculovirus expression system. Journal of General Virology 74 89-97.

RICHARDSON, J. C. & AKKINA, R. K. (1991) NS2 protein of influenza virus is found in purified virus and phosphorylated in infected cells. Archives of Virology 116, 69-80.

SCHLESINGER, J. J., BRANDRISS, M. W., CROPP, C. B. &MONATH, T. (1986) Protection against yellow fever virus in monkeys by immunization with yellow fever virus nonstructural protein NS1. Journal of Virology 60, 1153-1155.

SCHLESINGER, J. J., BRANDRISS, M. W. & WALSH, E. E. (1987) Protection of mice against dengue 2 virus encephalitis by immunization with the dengue 2 virus nonstructural glycoprotein NS 1. Journal of General Virology 68, 853-857.

SHAW, M. W., LAMON, E. W. & COMPANS, R. W. (1982) Immunologic studies on the influenza A virus nonstructural protein NS1. Journal of Experimental Medicine 156, 243-254.

SKEHEL, J. J. (1972) Polypeptide synthesis in influenza virus-infected cells. Virology 49, 23-36.

SMITH,D.B. & JOHNSON,K.S. (1988) Single-step purification of polypeptides expressed in Escherichia coli as fusions with glutathione S-transferase. Gene 67: 31-40.

SMITH, G. L., LEVIN, J. Z., PALESE, P. & MOSS, E. (1987) Synthesis and cellular location of the ten influenza polypeptides individually expressed by recombinant vaccinia virus. Virology 160, 336-345.

TOWBIN, H., STAEHELIN, T. & GORDON, J. (1979) Electrophoretic transfer of proteins from polyacrylamide gels to nitrocellulose sheets: procedure and some applications. Proceedings of the National Academy of Sciences of the United States of America 76, 4350-4354.

WOLSTENHOLME, A. J., BARRETT, T., NICHOL, S. T. & MAHBY, B. W. J. (1980) Influenza virus specific RNA and protein synthesis in cells infected with temperature sensitive mutants defective in the genome segment encoding nonstructural proteins. Journal of Virology 35, 1-7.

WOOD, J. M., MUMFORD, J. A., FOLKERS, C., SCOTT, A. M. & SCHILD, G. C. (1983) Studies with equine influenza vaccine: 1. Serological responses of ponies to graded doses of vaccine. Journal of Hygiene (Cambridge) 90, 371-384.

YOSHIDA, T., SHAW, M. W., YOUNG, J. F. & COMPANS, R. W. (1981) Characterization of the RNA associated with influenza A cytoplasmic inclusions and the interactions of NS 1 protein with RNA. Virology 110, 87-97.

YOUNG, J. F., DESSELBERGER, U., PALESE, P., FERGUSON, B., SCHATZMAN, A. R. & ROSENBERG, M. (1983) Efficient expression of influenza virus NS1 nonstructural proteins in Escherichia coli. Proceedings of the National Academy of Sciences of the United States of America 80, 6105-6109..pa

## Claims

1. Substantially pure recombinant non-structural protein NS1 obtainable from influenza virus A/equine 2/Suffolk 89 having the nucleotide sequence NS1 SEQ.ID.6 and functional homologues thereof by amino acid substitution, addition and/or deletion.

2. A protein comprising a portion of the protein of claim 1 having all or substantially all of the amino acid sequence selected from the group consisting of:
(i) Residues 1-128 of SEQ.ID.6 (NS1-N)
(ii) Residues 105-230 of SEQ.ID.6 (NS1-C).

3. A protein comprising a fusion of a protein according to claim 1 or 2 and glutathione S-transferase (GST).

4. An isolated DNA fragment containing a gene coding for a protein according to claims 1 or 2.

5. A vector containing in the correct reading frame an isolated DNA fragment according to claim 4.

6. A vector according to claim 5 which is derived from the plasmid group consisting of:
(i) pGEX-3X
(ii) pUC13.

7. An expression system comprising a host cell transformed with a vector according to claim 5 or 6.

8. A diagnostic assay for the determination of equine influenza A infection, which utilises a protein according to claim 1 or 2 in an screening step carried out on an equine serum sample to detect anti-NS1 antibody.

9. A diagnostic assay according to claim 8 in which the screening step is an ELISA.


SEQUENCE LISTING

(1) GENERAL INFORMATION:

(i) APPLICANT:
(A) NAME: Animal Health Trust
(B) STREET: PO Box 5
(C) CITY: Newmarket
(D) STATE: Suffolk
(E) COUNTRY: UK
(F) POSTAL CODE (ZIP): CB8 7DW
(G) TELEPHONE: (0638) 661111
(H) TELEFAX: (0638) 665789

(ii) TITLE OF INVENTION: EXPRESSION OF THE NON-STRUCTURAL PROTEIN NS1
OF INFLUENZA VIRUS AND DETECTION OF ANTI-NS1 ANTIBODY IN
SERUM

(iii) NUMBER OF SEQUENCES: 6

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 1:

CGGGATCCCC ATGGATTCCA ACACTGTGTC A 31

(2) INFORMATION FOR SEQ ID NO: 2:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 2:

CGGGATCCTC AAACTTCTGA CTCAATTGT 29

(2) INFORMATION FOR SEQ ID NO: 3:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 3:

CGGGATCCTC ATGCCCAAGC AG 22

(2) INFORMATION FOR SEQ ID NO: 4:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 4:

CGGGATCCTG ATGTTCTTAT CCA 23

(2) INFORMATION FOR SEQ ID NO: 5:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 5:

GCGACCATCC TCCAA 15

(2) INFORMATION FOR SEQ ID NO: 6:

(i) SEQUENCE CHARACTERISTICS:
(A) LENGTH: 693 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO: 6:

ATGGATTCCA ACACTGTGTC AAGCTTTCAG GTAGACTGTT TTCTTTGGCA TGTCCGCAAA 60

CGATTTGCAG ACCAAGAACT GGGTGATGCC CCATTCCTTG ACCGGCTTCG CCGAGACCAG 120

AAGTCCCTAA AAGGAAGAGG TAGCACTCTT GGTCTGGACA TCGAAACAGC CACTCGTGCA 180

GGAAAGCAGA TAGTGGAGCA GATTCTGGAA GAGGAATCAG ATGAGGCATT TAAAATGACC 240

ATTGCCTCTG TTCCTGCTTC ACGCTACTTA ACTGACATGA CTCTTGATGA GATGTCAAGA 300

GACTGGTTCA TGCTCATGCC CAAGCAGAAA GTAACAGGCT CCCTATGTAT AAGAATGGAC 360

CAGGCAATCA TGGATAAGAA CATCATACTT AAAGCAAACT TTAGTGTGAT TTTCGAAAGG 420

CTGGAGACAC TAATACTACT CAGGGCCTTC ACCGAAGAAG GAGCAGTCGT TGGCGAAATT 480

TCACCATTGC CTTCTCTTCC AGGACATACT AATGAGGATG TCAAAAATGC AATTGGGGTC 540

CTCATCGGAG GACTTAAATG GAATGATAAT ACGGTTAGAG TCTCTGAAAC TCTACAGAGA 600
TTCGCTTGGA GAAGCAGTCA TGAGAATGGG AGACCTTCAT TCCCTCCAAA GCAGAAACGA 660
AAAATGGAGA GAACAATTGA GTCAGAAGTT TGA 693